# EUROPEAN PATENT APPLICATION

(11) **EP 4 600 647 A1**
(43) Date of publication of application: **13.08.2025**
(21) Application number: 25155151.1
(22) Date of filing: 31.01.2025
(51) Int. Cl.: G01N 33/53, G01N 33/543, G01N 33/545, G01N 33/576

(54) **METHOD FOR PRODUCING PARTICLE FOR IMMUNOASSAY FOR DETECTING ANTI-HBS ANTIBODY, AND PARTICLE OBTAINED BY THE PRODUCTION METHOD**

(30) Priority: 06.02.2024 JP 2024016570; 16.01.2025 JP 2025006294
(71) Applicant: CANON KABUSHIKI KAISHA, Tokyo 146-8501 (JP)
(72) Inventor: KANAZAKI, Kengo, Tokyo (JP); YAMAUCHI, Fumio, Tokyo (JP); NATORI, Ryo, Tokyo (JP); KAWAMURA, Masashi, Tokyo (JP); ISHIOKA, Shuhei, Tokyo (JP); SUGIYAMA, Narumi, Tokyo (JP)
(74) Representative: WESER & Kollegen Patentanwälte PartmbB

(57) **Abstract**

The method for producing a particle for immunoassay for detecting an anti-HBs antibody, characterized by including a first step of covalently binding an HBs antigen to a particle to obtain an HBs antigen-sensitized particle, and a second step of washing the HBs antigen-sensitized particle with a nonionic surfactant or an amphoteric surfactant to obtain a washed HBs antigen-sensitized particle. Also, the method for producing a particle for immunoassay for detecting an anti-HBs antibody, characterized by including a first step of covalently binding an HBs antigen to a particle to obtain an HBs antigen-sensitized particle, and a third step of washing the HBs antigen-sensitized particle with an acidic aqueous solution to obtain a washed HBs antigen-sensitized particle.

## Description

### BACKGROUND OF THE INVENTION

### Field of the Invention

The present invention relates to a method for producing a particle for immunoassay for detecting an anti-HBs antibody, which is used in a latex immunoagglutination method, and a particle obtained by the production method.

### Description of the Related Art

About 400 million people worldwide are estimated to be carriers of hepatitis B virus (HBV). HBV infected people with the asymptomatic carrier state can progress to chronic hepatitis, and eventually hepatic cirrhosis. On the other hand, inactive carriers are known to have a low risk of disease stage progression and onset of cancer, and have a favorable long-term prognosis.

The fact that HBV replication in HBV infected patients occur due to immunosuppressive therapy, chemotherapy, or the like is called HBV reactivation. Hepatitis B is developed which is caused by HBV reactivation, among which fulminant cases may occur, and hence, caution is necessary. Therefore, when conducting immunosuppressive therapy and/or chemotherapy, it is necessary to measure the hepatitis B virus surface antigen (HBs antigen), hepatitis B virus core antigen (HBc) antibody and HBs antibody in all cases before starting the treatment so as to screen HBV infection.

The latex immunoagglutination method is used as one of the measuring methods for such an infection-related antigen or antibody. In this method, a dispersion of a particle obtained by sensitizing (binding) an antibody or an antigen as a ligand, and a specimen that may contain a substance to be measured (antigen or antibody) are mixed with each other. When a substance to be measured (antigen or antibody) is contained in the specimen, the particle that sensitized the ligand produces an agglutination reaction, and thus, by measuring this agglutination reaction as an amount of change in scattered light intensity, transmitted light intensity, absorbance, or the like, the determination of the presence or absence of and the quantification of the substance to be measured can be made.

With a latex agglutination test reagent for HBs antibodies, the measurement of the HBs antibody is performed using the particle that sensitized the HBs antigen as a ligand. The HBs antigen is an envelope protein that covers the surface of HBV, and there are known to be many variants such as subtype antigenic determinants d and y, w and r, and the like. As the HBs antigen to be used for the latex agglutination test reagent, an antigen purified from natural HBV can be used, or an antigen of a recombinant protein produced from Escherichia coli, cells or the like can be used. In HBV containing many variants, using an HBs antigen derived from natural HBV allows mutation variations of the HBs antigens to be reflected.

The HBs antigen is known to be a particle state presenting an antigenic protein on a lipid membrane. In Japanese Patent Application Laid-Open No. 2000-105233, making particle size smaller by a surfactant or the like to the HBs antigen in a particle state and causing the HBs antigen to react with a molecular chaperone are performed. The literature reported that treated HBs antigen thus obtained is then sensitized to a latex particle, whereby the detection sensitivity is enhanced. Also, Japanese Patent No. 4430677 reported a case where the detection sensitivity is enhanced by, after denaturing the HBs antigen using a denaturing agent or the like, exposing an epitope present inside and performing the detection using an antibody bound to the exposed epitope. Japanese Patent Application Laid-Open No. 2000-105233 and Japanese Patent No. 4430677 still had room for improvement in efficiency of sensitization to a latex particle.

### SUMMARY OF THE INVENTION

The method for producing a particle for immunoassay for detecting an anti-HBs antibody according to the present invention is characterized by including a first step of covalently binding an HBs antigen to a particle to obtain an HBs antigen-sensitized particle, and a second step of washing the HBs antigen-sensitized particle with a nonionic surfactant or an amphoteric surfactant to obtain a washed HBs antigen-sensitized particle.

The method for producing a particle for immunoassay for detecting an anti-HBs antibody according to the present invention is characterized by including the first step of covalently binding the HBs antigen to a particle to obtain an HBs antigen-sensitized particle, and a third step of washing the HBs antigen-sensitized particle with an acidic aqueous solution to obtain a washed HBs antigen-sensitized particle.

The particle for immunoassay for detecting an anti-HBs antibody according to the present invention is characterized in that the particle is produced by including a first step of covalently binding an HBs antigen to a particle to obtain an HBs antigen-sensitized particle, and a second step of washing the HBs antigen-sensitized particle with a nonionic surfactant or an amphoteric surfactant to obtain a washed HBs antigen-sensitized particle.

The particle for immunoassay for detecting an anti-HBs antibody according to the present invention is characterized in that the particle is produced by including a first step of covalently binding an HBs antigen to a particle to obtain an HBs antigen-sensitized particle, and a third step of washing the HBs antigen-sensitized particle with an acidic aqueous solution to obtain a washed HBs antigen-sensitized particle.

Further features of the present invention will become apparent from the following description of exemplary embodiments with reference to the attached drawing.

### BRIEF DESCRIPTION OF THE DRAWINGS

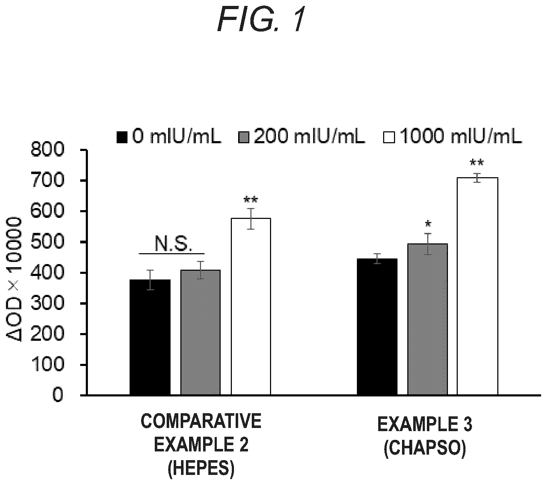

FIG. 1 illustrates the measurement results of the detection sensitivity of anti-HBs of the particle for immunoassay for detecting an anti-HBs antibody produced by the production method according to the present invention.

### DESCRIPTION OF THE EMBODIMENTS

Preferred embodiments of the present invention will now be described in detail in accordance with the accompanying drawing.

Hereinafter, embodiments of the present invention will be described in detail; however, the technological scope of the present invention is not limited to the following embodiments.

### (Description of the Object)

Problems to be solved by the method for producing a particle according to embodiments of the present invention will be described. The HBs antigen derived from natural HBV is collected and purified from a living body. Therefore, the HBs antigen derived from natural HBV contains impurities such as lipids and unnecessary proteins. The fact that there are the impurities was one of the reasons that hindered the HBs antigen and the anti-HBs antibody from binding and caused the sensitivity of latex agglutination reaction to be decreased.

As described in Japanese Patent Application Laid-Open No. 2000-105233 and Japanese Patent No. 4430677, approaches to making particle size smaller by treating the HBs antigen in advance with a surfactant or the like, or to removing impurities have been proposed. However, the HBs antigen premixed with a surfactant has a reduced efficiency of sensitization to a latex particle having a functional group (e.g., carboxy group), which poses a problem. Also, since some surfactants and reductants disclosed in Japanese Patent No. 4430677 destroy the nearby structure of the antigenic determinant a, which is a main conformational epitope of the HBs antigen, there has been a problem in that these surfactants and reductants could not be used as a reagent for treating the HBs antigen used in the anti-HBs antibody test.

### (Production Method)

The method for producing a particle according to embodiments of the present invention will be described. The method for producing a particle for immunoassay for detecting an anti-HBs antibody according to the present invention is characterized by including a first step of covalently binding an HBs antigen to a particle to obtain an HBs antigen-sensitized particle, and a second step of washing the HBs antigen-sensitized particle with a nonionic surfactant or an amphoteric surfactant to obtain a washed HBs antigen-sensitized particle.

Here, as the covalent bond in the first step, any bond can be used as long as the HBs antigen can be bound to the functional group of the particle. An amino group, a carboxy group, a thiol group and the like are present in the HBs antigen, any functional group of the particle can be used as long as the functional group can react with these groups.

Examples of the functional group that reacts with the amino group of the HBs antigen to form a covalent bond include N-hydroxysuccinimide ester, sulfo-N-hydroxysuccinimide ester, isothiocyanate, isocyanate, acyl azide, sulfonyl chloride, aldehyde, glyoxal, epoxide, oxirane, carbonate, aryl halide, imidoester, carbodiimide, anhydride, and fluoro ester. Of these, N-hydroxysuccinimide ester and sulfo-N-hydroxysuccinimide ester in which the reaction efficiently proceeds even at around neutral pH are preferable. The particle having a carboxy group as the functional group is preferable because the particle can introduce N-hydroxysuccinimide ester or sulfo-N-hydroxysuccinimide ester via activation of carbodiimide.

Examples of the functional group that reacts with the carboxy group of the HBs antigen to form a covalent bond include the amino group. In this case, the carboxy group of the HBs antigen can introduce N-hydroxysuccinimide ester or sulfo-N-hydroxysuccinimide ester via activation of carbodiimide and then can bind to the amino group of the particle.

Examples of the functional group that reacts with the thiol group of the HBs antigen to form a covalent bond include the maleimide group and the sulfide group. Note that, in the present invention, the particle obtained by covalently binding with the HBs antigen is referred to as the HBs antigen-sensitized particle.

Next, as the nonionic surfactant or amphoteric surfactant in the second step, any bond can be used as long as the favorable washing effect can be obtained.

Here, as shown in Examples described later, a positive surfactant is not preferable because the particle surface bearing negative charge and the positive surfactant having positive charge interact with each other to cause the particle to be hydrophobic, and the agglutination occurs due to the hydrophobic interaction, whereby it is difficult to use the positive surfactant as the test reagent.

Further, as shown in Examples described later, a negative surfactant is not preferable because the particle surface bears negative charge, and thus the surfactant does not easily approach the particle, and the reaction to remove impurities tends not to proceed.

A living body-derived component contained in a patient specimen or the like is likely to be negatively charged at around neutral pH. In order to suppress a non-specific reaction with such a living body-derived component, the majority of the particles used in a specimen test have a particle surface negatively charged. In other words, the positive and negative surfactants are not preferable as the surfactant in the method for producing a particle according to the embodiments of the present invention. On the other hand, the nonionic surfactant and amphoteric surfactant are unlikely to be affected by the charge on the particle surface, and therefore, these surfactants can obtain a high washing effect. Note that, in the present invention, the particle obtained by washing the HBs antigen-sensitized particle with the nonionic surfactant or amphoteric surfactant is referred to as the washed HBs antigen-sensitized particle.

Here, the washing and replacing operation means an operation of mixing the nonionic surfactant or amphoteric surfactant with the HBs antigen-sensitized particle, and after a certain time passed, replacing the solution with a buffer solution containing no surfactant. The duration, temperature and with or without shaking in mixing can be arbitrarily set as long as the washing effect can be obtained. For example, when the mixing operation is performed for 22 hours, at room temperature (20°C to 25°C), and with shaking (microtube mixer MT-400 (manufactured by Tomy Seiko Co., Ltd.), at scale 5), a high washing effect can be obtained. The operation of replacing the solution with a buffer solution containing no surfactant can be performed by dialysis using a dialysis membrane, cross flow using an ultrafiltration membrane, centrifugation, or the like. Note that, in the present invention, the replacing the solution with a buffer solution is referred to as a fourth step.

The method for producing a particle for immunoassay for detecting an anti-HBs antibody according to the present embodiment of the present invention needs to wash the HBs antigen-sensitized particle obtained in the first step with a nonionic surfactant or an amphoteric surfactant in the second step, or need to wash the HBs antigen-sensitized particle obtained in the first step with an acidic aqueous solution in the third step. Here, the second and third steps can be performed after the first step. As shown in Examples described later, if the second step is performed first, the amount of the HBs antigen sensitized was significantly reduced, and the detection sensitivity of the anti-HBs antibody was also significantly reduced. The reason is considered that the surfactant slightly remained even after the dialysis and purification were thoroughly performed in the second step hinders the sensitization reaction with the particle.

Another method for producing a particle for immunoassay for detecting an anti-HBs antibody according to the present invention includes the first step of covalently binding the HBs antigen to a particle, and a third step of washing the particle with an acidic aqueous solution.

Here, as shown in Examples described later, an alkali aqueous solution is not preferable because the solution causes the HBs antigen of the HBs antigen-sensitized particle to be partially dissociated or denatured, and causes a decreased amount sensitized and decreased detection sensitivity of the anti-HBs antibody. Also, the reductant is not preferable because the reductant destroys the nearby structure of the antigenic determinant a, which is a main conformational epitope of the HBs antigen of the HBs antigen-sensitized particle, and causes decreased detection sensitivity of the anti-HBs antibody. On the other hand, the acidic aqueous solution can obtain a high washing effect without having large denaturation of the HBs antigen. Note that, in the present invention, the particle obtained by washing the HBs antigen-sensitized particle with the acidic aqueous solution is also referred to as the washed HBs antigen-sensitized particle.

### (Surfactant)

As the surfactant in the production method according to the present embodiment, a nonionic surfactant or an amphoteric surfactant can be used. Examples of the nonionic surfactant include polyoxyethylene sorbitan-based fatty acid ester (e.g., compound represented by the formula (1)), Brij (registered trademark) 35, Brij (registered trademark) 58, Brij (registered trademark) 76, Brij (registered trademark) 98, Triton (registered trademark) X-100, Triton (registered trademark) X-114, Triton (registered trademark) X-305, Triton (registered trademark) N-101, Nonidet (registered trademark) P-40, IGEPAL (registered trademark) CO530, IGEPAL (registered trademark) CO630, IGEPAL (registered trademark) CO720 and IGEPAL (registered trademark) CO730. 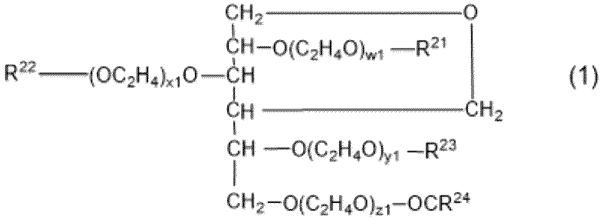 (in the formula (1), R²¹ to R²⁴ are each independently selected from -H and -OCR'. The R' is a saturated or unsaturated alkyl group having 1 or more and 18 or less carbon atoms. In addition, in the formula (1), w1, x1, y1 and z1 are integers such that the total sum of w1, x1, y1 and z1 is 10 or larger and 30 or smaller.)

Examples of the polyoxyethylene sorbitan-based fatty acid ester represented by the formula (1) include Tween (registered trademark) 20, Tween (registered trademark) 40, Tween (registered trademark) 60, Tween (registered trademark) 80 and Tween (registered trademark) 85.

Examples of the amphoteric surfactant in the production method according to the present embodiment include a betaine type, an alkylbetaine type and a sulfobetaine type. Here, the amphoteric surfactant is a surfactant in which a portion of the hydrophilic group changes its state from being positively charged to negatively charged and vice versa, depending on the pH.

### (Acidic Aqueous Solution)

As the acidic aqueous solution in the production method according to the present embodiment, an aqueous solution of hydrochloric acid, an aqueous solution of acetic acid, an aqueous solution of glycine hydrochloride, or the like can be used. The acidic aqueous solution is preferably used at a concentration ranging from 0.01 to 2M. If the acidity of the acidic aqueous solution is too strong, the HBs antigen is acid-denatured and the binding properties to the anti-HBs antibody is decreased, which is not preferable. On the other hand, if the acidity of the acidic aqueous solution is too weak, a sufficient washing effect cannot be obtained.

### (Particle)

The particle in the production method according to the present embodiment is a particle used in a general immunological test, and any particle can be used as long as the particle has a functional group that can covalently bind to HBs. Examples of the material for the particle include polymers such as polystyrene, styrene-methacrylic acid copolymer, styrene-glycidyl (meth)acrylate copolymer, acrylic acid copolymer, agarose and dextran, inorganic substances such as silica and alumina, and metals such as gold colloid, iron oxide particle and magnetic particle. Particle synthesis and preparation can be performed according to a known method.

Examples of the suitable particle include a latex particle. Here, the latex particle is a polymer particle capable of immobilizing an antigen or antibody against a target substance, and refers to a particle used in a latex agglutination reaction. Preferred examples of the latex particle include a polystyrene particle, a polystyrene particle containing siloxane and a polystyrene particle containing polyglycidyl (meth)acrylate. These particles have advantages such as the fact that a nano-sized particle can be obtained relatively easily and a surface of each particle can be chemically modified according to the purpose, and is a particle that can be suitably used with the latex agglutination reagent of the present embodiment.

Among these particles, as will be described later in Examples, a particle, especially styrene-glycidyl (meth)acrylate copolymer, in which one or more glycidyl groups are ring-opened with mercapto succinate and 3-mercapto-1,2-propanediol has a chemically hydrophilic particle surface, and can highly suppress non-specific adsorption.

In addition, among the polystyrene particles containing siloxane, as will be described later in Examples, a polystyrene-silica hybrid particle with 3-methacryloxypropyltrimethoxysilane used as a monomer, also has a hydrophilic particle surface, and can highly suppress non-specific adsorption.

The particle size is preferably 0.01 µm or greater and 10 µm or smaller, and more preferably 0.05 µm or greater and 1 µm or smaller, as the long axis diameter. When the particle size is set to 0.01 µm or greater, the absorbance in the visible range observed by the latex agglutination measurement is within an appropriate range. When the particle size is set to 10 µm or smaller, the dispersion stability in the solution is improved, and the absorbance in the visible range is within an appropriate range. From the viewpoint of reactivity and dispersion stability, a particle of 0.05 µm or greater and 0.50 µm or smaller is preferably used. A suitable example of the size can be represented in terms of average particle size.

As the average particle size, a volume average particle size or the like measured by the measurement of scattered particle size distribution is used.

The average particle size of the particle is preferably 0.05 µm or greater and 1 µm or smaller.

### (HBs Antigen)

As the HBs antigen in the production method according to the present embodiment, HBV collected from blood of hepatitis B patients (or Dane particle) can be inactivated for use. For example, a virus is inactivated by subjecting to liquid heat treatment at 60°C for 10 hours, and then, by a concentration gradient centrifugation purification method using sucrose or potassium bromide or gel filtration purification, the HBs antigen can be obtained.

Further, an HBs antigen prepared by gene recombination can also be used. HBs can be artificially produced by introducing a gene that encodes HBs into Escherichia coli or mammalian cells. Since the HBs prepared by gene recombination contains the amino group determined based on the genetic information, mutation variations can be possibly reduced. Accordingly, ingenuity such as using multiple genetic information is required.

### (Binding between HBs Antigen and Particle)

In the present embodiment, to the chemical reaction method in which the carboxy group contained in the particle according to the present embodiment reacts with the amino group of the HBs antigen to form an amide bond, and HBs is chemically immobilized, a conventionally known method can be applied as long as the object of the present invention is within an achievable range. For example, a carbodiimide-mediated reaction and an NHS ester activation reaction are commonly used chemical reactions.

However, in the present invention, the chemical reaction method for chemically immobilizing the carboxy group and HBs is not limited to these reactions.

In the HBs-sensitized particle in the present embodiment, a hydrophilic molecule can be bound to the remaining active-esterified carboxy group to which HBs is not bound. This is generally referred to as active ester inactivation, carboxy group blocking treatment or masking treatment, and is performed to reduce non-specific adsorption of proteins to carboxy group, and improve the dispersion stability of the particle. In the present embodiment, the above-mentioned hydrophilic molecule is preferably polyethylene glycol (PEG), tris(hydroxymethyl)aminomethane (Tris) or ethanol amine. PEG can greatly reduce adsorption of proteins to the particle. When the active ester inactivation is performed using PEG, the molecular weight of PEG is important; if the molecular weight is large, the antigen-antibody reaction may be hindered. For this reason, the molecular weight of PEG is preferably 350 or more and 5000 or less, and particularly preferably 1000 or more and 2000 or less. As PEG in the present embodiment, PEG having a carboxy group or a functional group reactive with an active ester, for example, PEG having an amino group is preferable, and polyethylene glycol having a primary amine is particularly preferable. Polyethylene glycol may be a linear polymer or a branched polymer. Tris is represented by the following formula (2), and examples of PEG are represented by the following formulas (3) and (4). Note that, n in the formulas (3) and (4) is an integer of 1 or more, indicating the number of oxyethylene units.

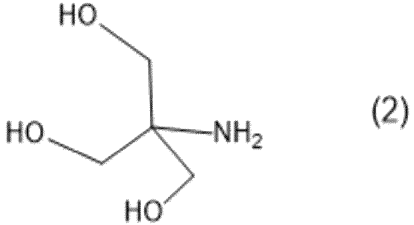

CH₃O-(CH₂CH₂O)n-CH₂CH₂NH₂ (3)

CH₃O-(CH₂CH₂O)n-CH₂CH₂CH₂NH₂ (4)

The amount of binding HBs antigen is also an important factor; if the amount of HBs antigen bound (may be referred to as the amount of immobilizing) is small, the reactivity of the antigen-antibody decreases, whereas antibody binding such that particles are cross-linked to each other is likely to occur. Contrary to this, if the amount of HBs antigen bound is large, the reactivity of the antigen-antibody improves, but 2 binding sites of antigens and antibodies on one particle are easily bound to each other, and cross-linking of particles is unlikely to occur. Although it depends on the particle size, if the average particle size is about 200 nm, the amount of HBs antigen bound is preferable to be 1 µg or more and 500 µg or less per 1 mg of particle, and particularly preferable to be 10 µg or more and 200 µg or less.

### (Test Reagent)

The in vitro diagnostic test reagent according to the present embodiment contains the particle for immunoassay for detecting an anti-HBs antibody according to the present embodiment and a dispersion medium. The amount of the particle for immunoassay for detecting an anti-HBs antibody contained in the test reagent in the present embodiment is preferably 0.001% by mass to 20% by mass, and more preferably 0.01% by mass to 10% by mass. The reagent of the present invention may contain a third substance such as a solvent or a blocking agent in addition to the particle for immunoassay for detecting an anti-HBs antibody according to the present embodiment, as long as the object of the present invention is within an achievable range. The third substance such as a solvent or a blocking agent can be contained in combination of two or more. Examples of the solvent used in the present invention include various buffer solutions such as phosphate buffer solution, glycine buffer solution, Good's buffer solution, Tris buffer solution and ammonia buffer solution; however, the solvent contained in the reagent according to the present embodiment is not limited to these.

The in vitro diagnostic test kit according to the present embodiment contains the test reagent according to the present embodiment and a housing enclosing the test reagent. The test kit according to the present embodiment preferably further contains a reaction buffer containing albumin (hereinafter, reagent 2) in addition to the test reagent according to the present embodiment (hereinafter, reagent 1). Examples of the albumin include serum albumin, which may be protease-treated. The amount of albumin contained in the reagent 2 is targeted to be 0.001% by mass to 5% by mass; however, the test kit according to the present embodiment is not limited to these. Both or either of the reagent 1 and reagent 2 may contain a sensitizer for latex immunoagglutination measurement. Examples of the sensitizer for latex immunoagglutination measurement include, but is not limited to, polyethylene glycol, polyvinyl alcohol, polyvinylpyrrolidone and polyalginic acid. Both or either of the reagent 1 and reagent 2 may contain a surfactant. Since the surfactant has an effect of stabilizing a particle or a protein, for example, polyoxyethylene sorbitan monolaurate or poly(oxyethylene) octylphenyl ether are preferably used. In addition, the test kit in the present embodiment may contain a positive control, a negative control, serum diluent or the like, in addition to the reagent 1 and reagent 2. As a medium for the positive control and/or negative control, serum that does not contain a measurable target substance, physiological saline or a solvent can be used.

### (Detection Method)

The method for detecting a target substance in the specimen by the latex immunoagglutination method of the present embodiment is characterized by mixing the particle for immunoassay for detecting an anti-HBs antibody of the present embodiment with a specimen that may contain the target substance. Also, the mixing of the particle for immunoassay for detecting an anti-HBs antibody of the present embodiment and the specimen is preferably performed within the range of pH 3.0 to pH 11.0. Further, the mixing temperature is within the range of 20°C to 50°C, and the mixing time is within the range of 10 seconds to 30 minutes. In addition, the concentration of the particle for immunoassay for detecting an anti-HBs antibody of the present embodiment in the detection method of the present embodiment is preferably 0.001% by mass to 5% by mass, and more preferably 0.01% by mass to 1% by mass, in a reaction system. The detection method of the present embodiment optically detects the agglutination reaction occurred as a result of the mixing of the particle for immunoassay for detecting an anti-HBs antibody of the present embodiment and the specimen. Specifically, by optically detecting the agglutination reaction, the target substance in the specimen is detected, and further, the concentration of the target substance can also be measured. As the method for optically detecting the agglutination reaction, an optical apparatus capable of detecting the scattered light intensity, transmitted light intensity, absorbance, or the like can be used to measure the amount of change in these values.

### (Other Immunological Test Reagent)

The particle prepared by the production method according to the embodiments of the present invention can also be used for an immunological test reagent in a method other than the latex immunoagglutination method. Examples thereof include, but is not limited to, an immunochromatogram method, a chemiluminescent enzyme immunoassay method, an electrochemiluminescent immunoassay method and enzyme immunoassay (including competitive method).

### [Examples]

Hereinafter, the present invention will be described in more detail by reference to Examples; however, the present invention is not limited to these Examples.

### (Example 1: Particle Synthesis)

Into a 2L 4-necked separable flask, 12.0 g styrene (St; manufactured by Kishida Chemical Co., Ltd.), 17.9 g glycidyl methacrylate (GMA; Tokyo Chemical Industry Co., Ltd.), 0.45 g divinylbenzene (DVB; Kishida Chemical Co., Ltd.) and 2168.6 g ion exchanged water were weighed and loaded to make a mixed solution. The solution was then maintained at 70°C, while stirring at 200 rpm, and the nitrogen flow was performed at a flow rate of 200 ml/min to deoxygenate the above 4-necked separable flask. Next, a dissolution solution separately prepared by dissolving 1.13 g V-50 (FUJIFILM Wako Pure Chemical Corporation) into 30 g ion exchanged water was added to the mixed solution to start soap-free emulsion polymerization. 2 hours after the start of the polymerization, 3.1 g GMA was added to the 4-necked separable flask, and the mixture was maintained for further 22 hours at 70°C while stirring at 220 rpm to obtain an aqueous dispersion containing a granular copolymer A. After slowly cooling the dispersion to room temperature, a part thereof was collected, and the polymerization conversion rate was evaluated using proton NMR, gas chromatography and gel permeation chromatography, and thereby it was confirmed to be substantially 100%. The particle size in dry state and the particle size in water of the granular copolymer A were 151.4 nm and 160.2 nm, respectively. The granular copolymer A was subjected to ultrafiltration concentration or diluted by adding ion exchanged water so as to be an aqueous dispersion of 2.5 w/v%, and stored at 4°C in the shading condition. Next, 2.5 w/v% aqueous dispersion containing 24 g granular copolymer A, 3.3 g ion exchanged water, 40 mg (0.26 mmol) mercapto succinate (FUJIFILM Wako Pure Chemical Corporation) and 0.214 mL (2.34 mmol) 3-mercapto-1,2-propanediol (FUJIFILM Wako Pure Chemical Corporation) were weighed and loaded into 100 ml round-bottom flask, and triethylamine (Kishida Chemical Co., Ltd.) was added thereto to adjust to pH = 10. Next, the contents in the round-bottom flask were heated to 70°C while stirring at 200 rpm and maintained in this state for further 18 hours to obtain a particle dispersion. The particle was separated from the dispersion using a centrifuge, and further, the operation of redispersing the particle in ion exchanged water was repeated 8 times to purify the particle, and eventually, the aqueous dispersion was adjusted to a particle concentration of 1.0 w/v% and stored in that state. The storage condition was set to 4°C in the shading condition.

### (Example 2: Sensitization of HBs Antigen)

0.1 mL (1 mg particle) each of the particle dispersion (solution with concentration of 1.0% by mass) prepared in Example 1 was transferred to a microtube (1.5 mL volume). Into this, 0.12 mL activating buffer solution (25 mM MES buffer solution, pH 6.0) was added, and the mixture was centrifuged at 4°C, 15000 rpm (20400 g) for 5 minutes. After the centrifugation, the supernatant was discarded using a pipetter. Subsequently, 0.12 mL activating buffer solution was added thereto, and the mixture was dispersed by ultrasonication using an ultrasonic cleaner (trade name: MODEL VS-100III As One 3-frequency ultrasonic cleaner, manufactured by As One Corporation, 28 kHz). Next, the mixture was centrifuged at 4°C, 15000 rpm (20400 g) for 5 minutes. The supernatant was discarded using a pipetter, and 0.12 mL activating buffer solution was added to disperse the mixture by ultrasonication. Subsequently, the mixture was centrifuged at 4°C, 15000 rpm (20400 g) for 5 minutes, and the supernatant was discarded using a pipetter. Subsequently, 60 µL each of WSC solution (obtained by dissolving 50 mg WSC in 1 mL activating buffer solution) and N-hydroxysulfosuccinimide (Sulfo NHS) solution (obtained by dissolving 50 mg Sulfo NHS in 1 mL activating buffer solution) was added thereto. After the addition, the mixture was dispersed by ultrasonication. By further stirring the mixture at room temperature for 30 minutes, the carboxy group of the particle was converted into an active ester.

Next, the dispersion was centrifuged at 4°C, 15000 rpm (20400 g) for 5 minutes, and the supernatant was discarded using a pipetter. 0.2 mL immobilizing buffer solution (25 mM MES buffer solution, pH 4.0) was added to disperse the mixture by ultrasonication. The mixture was centrifuged at 4°C, 15000 rpm (20400 g) for 5 minutes, and the supernatant was discarded using a pipetter. 50 µL immobilizing buffer solution per 1 mg of particle was added to disperse the particle in which the carboxy group was activated by ultrasonication.

The HBs antigen (manufactured by Institute of Immunology Co., Ltd.) was diluted by an immobilizing buffer solution so as to be 100 µg/50 µL. 50 µL solution of HBs antigen was added to 50 µL solution of the particle (containing 1 mg particle) in which the carboxy group was activated, to disperse the particle by ultrasonication. The amount of HBs antigen to be charged will be 100 µg per 1 mg of the particle (100 µg/mg). The tube was stirred at 23°C for 210 minutes to immobilize the antigen to the carboxy group of the particle. Next, the mixture was centrifuged at 4°C, 15000 rpm (20400 g) for 5 minutes, and the supernatant was discarded using a pipetter.

0.24 mL active ester inactivating buffer solution containing tris(hydroxymethyl)aminomethane (Tris) (Tris buffer solution, pH 8.0 including 0.1% Tween (registered trademark) 20) was added to disperse the mixture by ultrasonication. The mixture was stirred at room temperature for 1 hour, Tris was bound to the remaining active ester, and then, the mixture was left to stand overnight at 4°C.

The mixture was then centrifuged at 4°C, 15000 rpm (20400 g) for 5 minutes, and the supernatant was discarded using a pipetter. 0.2 mL washing and storing buffer solution (10 mM 2-[4-(2-hydroxyethyl)-1-piperazinyl]-ethanesulfonic acid (HEPES) buffer solution, pH 7.9) was added to disperse the mixture by ultrasonication. The washing operation by 0.2 mL washing and storing buffer solution was repeated twice, and 1.0 mL washing and storing buffer solution was then added to disperse the mixture by ultrasonication. During the above process, loss of the particle was hardly observed, and therefore, the final concentration of the HBs antigen-sensitized particle was 0.6% by mass (6 mg/mL). The mixture was stored in the refrigerator and redispersed by ultrasonication in use.

### (Example 3: Processing Operation with Surfactant or the Like)

0.166 mL (1 mg) HBs antigen-sensitized particle prepared in Example 2, and 0.05 mL aqueous solution of 10% dodecyl-β-D-maltoside, 0.05 mL aqueous solution of 10% Triton (registered trademark) X-100 or 0.05 mL aqueous solution of 10% 3-[(3-cholamidopropyl)dimethylammonio]-2-hydroxy-1-propanesulfonate (CHAPSO), and 0.034 mL 10 mM HEPES buffer solution (pH 7.9) were mixed and shook for 22 hours at room temperature (20°C to 25°C) with a microtube mixer MT-400 (manufactured by Tomy Seiko Co., Ltd.) at scale 5, to obtain a solution containing a washed HBs antigen-sensitized particle.

The particle solution after the reaction was washed three times with 0.25 mL HEPES buffer solution (pH 7.9), and eventually redispersed in 0.33 mL HEPES buffer solution (pH 7.9) containing 0.01% Tween (registered trademark) 20 to obtain a solution containing a particle for immunoassay for detecting an anti-HBs antibody.

### (Example 4: Processing Operation with Acidic Aqueous Solution)

0.166 mL (1 mg) HBs antigen-sensitized particle prepared in Example 2, and 0.084 mL aqueous solution of 1M hydrochloride were mixed and shook for 22 hours at room temperature (20°C to 25°C) with a microtube mixer MT-400 (manufactured by Tomy Seiko Co., Ltd.) at scale 5, to obtain a solution containing a washed HBs antigen-sensitized particle.

The particle solution after the reaction was washed three times with 0.25 mL HEPES buffer solution (pH 7.9), and eventually redispersed in 0.33 mL HEPES buffer solution (pH 7.9) containing 0.01% Tween (registered trademark) 20 to obtain a solution containing a particle for immunoassay for detecting an anti-HBs antibody.

### (Comparative Example 1)

Preparation was performed as in Example 3 except that 0.05 mL aqueous solution of 10% sodium N-dodecanoylsarcosinate or 0.05 mL aqueous solution of 10% dodecyltrimethylammonium chloride was used in place of various surfactants used in Example 3, and surfactant treatment was performed to obtain a particle for immunoassay for detecting an anti-HBs antibody.

### (Comparative Example 2)

Preparation was performed as in Example 4 except that 0.084 mL aqueous solution of 1M sodium hydroxide, 0.084 mL aqueous solution of 0.1 M dithiothreitol or 0.84 mL 10 mM HEPES buffer solution (pH 7.9) was used in place of the aqueous solution of hydrochloric acid used in Example 4 to obtain a particle for immunoassay for detecting an anti-HBs antibody.

The particle size of the thus obtained particle for immunoassay for detecting an anti-HBs antibody was evaluated by dynamic light scattering (DLS). Zetasizer Nano ZS (manufactured by Malvern Panalytical Ltd.) was used for measurements. The measured volume average particle size and poly dispersity index are shown in Table 1.

The HBs antigen was confirmed to have been sensitized (immobilized) to the particle through protein quantification. Specifically, this is a method of reacting the particle for immunoassay for detecting an anti-HBs antibody with a BCA reagent. First, 25 µL (amount of particle: 25 µg) of the dispersion (0.1% solution) of the particle for immunoassay for detecting an anti-HBs antibody was taken out. 7 mL of solution A and 140 µL of solution B of the protein assay BCA kit (Wako Pure Chemical Industries, Ltd.) were mixed to prepare a solution AB. 200 µL solution AB was added to the particle solution (25 µL), and the mixture was incubated for 30 minutes at 60°C.

The solution was centrifuged at 4°C, 15000 rpm (20400 g) for 5 minutes, and 200 µL supernatant was collected using a pipetter. The absorbance thereof was measured at 562 nm, along with standard samples (several samples of HBs antigen in 10 mM HEPES within 0 to 200 µg/mL) with a multimode microplate reader (SynergyMX, BioTek). The amount of HBs antigen was calculated from the standard curve. The amount of HBs antigen sensitized to the particle (amount of HBs antigen bound (amount of HBs antigen immobilized) per particle weight (µg/mg)) was determined by dividing the calculated amount of HBs antigen by the particle weight (here, 0.025 mg). The measured amounts of HBs antigen sensitized are shown in Table 1.

**[Table 1]**

| | Item | Name | Volume average particle size (nm) | Poly dispersity index | Amount sensitized per 1 mg of particle (µg/mg) |
|---|---|---|---|---|---|
| Example 3 | Nonionic surfactant | Dodecyl-β-D-maltoside | 272.1 | 0.006 | 82 |
| | Nonionic surfactant | Triton(registered trademark)X-100 | 270.4 | 0.027 | 78 |
| | Amphoteric surfactant | 3-[(3-cholamidopropyl)dimethylammonio]-2-hydroxy-1-propanesulfonate | 275.7 | 0.024 | 78 |
| Example 4 | Acidic aqueous solution | Hydrochloric acid | 271.2 | 0.041 | 80 |
| Comparative example 1 | Negative surfactant | Sodium N-dodecanoylsarcosinate | 284.7 | 0.042 | 77 |
| | Positive surfactant | Dodecyltrimethylammonium chloride | 431.8 | 0.119 | 71 |
| Comparative example 2 | Alkali aqueous solution | Sodium hydroxide | 284.8 | 0.025 | 40 |
| | Reductant | Dithiothreitol | 310.5 | 0.104 | 84 |
| | Reference | HEPES buffer solution | 300.6 | 0.091 | 77 |

### (Example 5: Evaluation of Sensitivity to Anti-HBs Antibody)

The sensitivity of the particle for immunoassay for detecting an anti-HBs antibody according to the present invention was evaluated by the latex immunoagglutination method. Specifically, this is a method in which the particle for immunoassay for detecting an anti-HBs antibody is reacted with the anti-HBs antibody to form aggregates of immune complex, the aggregates are irradiated with light, and the attenuation (absorbance) of the irradiated light due to scattering is measured using an absorption spectrometer. Depending on the amount of antigen contained in the specimen, the proportion of the aggregates increases, and the absorbance increases. In the evaluation of sensitivity, it is desirable that the increased amount of the absorbance (expressed as ΔOD × 10000) at a predetermined concentration of anti-HBs antibody be large. An ultraviolet-visible spectrophotometer (BioSpectrometer kinetic (manufactured by Eppendorf GmbH)) was used for measuring the absorbance, and the samples were injected into a plastic cell and measured at an optical path length of 2 mm. The specific measurement method is shown below.

Specifically, 1 µL solution of anti-HBs mouse antibody (concentration of anti-HBs mouse antibody at 0 mg/mL or 0.1 mg/mL) was used as a sample, and the sample and 60 µL buffer solution for dilution (PBS, 0.01% Tween (registered trademark) 20) were mixed in a plastic cell and heated at 37°C for 5 minutes. 30 µL dispersion solution of the particle for immunoassay for detecting an anti-HBs antibody (particle concentration of 0.3 wt%, 10 mM HEPES, pH 7.9, 0.01 wt% Tween (registered trademark) 20) was added to the buffer solution for dilution (61 µL) containing anti-HBs mouse antibody, and the sample was pipetted quickly with caution so as not to introduce air bubbles. The absorbance of the sample at 572 nm was read and designated as Abs 1. The sample was heated at 37°C for 5 minutes, and absorbance of the sample at 572 nm was then read and designated as Abs 2. The value obtained by subtracting Abs 1 from Abs 2 was calculated and multiplied by 10000 to obtain a ΔOD × 10000 value.

The results are shown in Table 2. In the particle for immunoassay for detecting an anti-HBs antibody of this Example, an increase in ΔOD × 10000 was observed in the presence of the anti-HBs mouse antibody. This is the result of the particle for immunoassay for detecting an anti-HBs antibody binding to the anti-HBs mouse antibody to form particle aggregates, which were found to function as a particle for use in the latex immunoagglutination method. Further, the ΔOD × 10000 values of the particles for immunoassay for detecting an anti-HBs antibody prepared in Examples 3 and 4 were found to be larger than those of the particles for immunoassay for detecting an anti-HBs antibody prepared in Comparative Examples 1 and 2, indicating the effectiveness of the production method of the present invention.

**[Table 2]**

| | Item | Name | ΔOD × 10000 | | Increase/decrease rate to reference at anti-HBs antibody concentration of 0.1 mg/mL |
|---|---|---|---|---|---|
| | | | Anti-HBs antibody concentration of 0 mg/mL | Anti-HBs antibody concentration of 0.1 mg/mL | |
| Example 3 | Nonionic surfactant | Dodecyl-β-D-maltoside | -420 | 25600 | 1.2 |
| | Nonionic surfactant | Triton(registered trademark)X-100 | -590 | 26350 | 1.2 |
| | Amphoteric surfactant | 3-[(3-cholamidopropyl)dimethylammonio]-2-hydroxy-1-propanesulfonate | -200 | 26280 | 1.2 |
| Example 4 | Acidic aqueous solution | Hydrochloric acid | -270 | 25750 | 1.2 |
| Comparative example 1 | Negative surfactant | Sodium N-dodecanoylsarcosinate | 130 | 21560 | 1 |
| | Positive surfactant | Dodecyltrimethylammonium chloride | -680 | 17160 | 0.8 |
| Comparative example 2 | Alkali aqueous solution | Sodium hydroxide | 210 | 10 | o |
| | Reductant | Dithiothreitol | 20 | -410 | 0 |
| | Reference | HEPES buffer solution | -400 | 21640 | 1 |

As can be seen in Table 1, the particle size of the particle for immunoassay for detecting an anti-HBs antibody treated by the positive surfactant (dodecyltrimethylammonium chloride) became bigger. The reason is considered that the particle surface bearing negative charge and the positive surfactant having positive charge interacted with each other to cause the particle to be hydrophobic, the agglutination occurred due to the hydrophobic interaction.

The particle size nor ΔOD × 10000 values of the particle for immunoassay for detecting an anti-HBs antibody treated by the negative surfactant (N-sodium N-dodecanoylsarcosinate) changed from the reference (treated by 10 mM HEPES buffer solution (pH 7.9)). The reason is considered that the particle surface boar negative charge, and thus the surfactant did not easily approach the particle, the reaction to remove impurities did not proceed.

From the above reasons, it is considered that the positive surfactant and negative surfactant did not obtain the effects of the present invention even mixed with the HBs antigen-sensitized particle.

On the other hand, the nonionic surfactant and amphoteric surfactant had slightly smaller particle size (Table 1) and increased ΔOD × 10000 values (Table 2) compared to the reference. The reason is considered that these surfactants suitably reacted to the HBs antigen-sensitized particle sensitized on the particle surface and exerted the effect of removing impurities, and their sensitivity improved.

As can be seen in Table 1, the amount sensitized of the particle for immunoassay for detecting an anti-HBs antibody treated by the alkali aqueous solution (sodium hydroxide) decreased. The reason is considered that a part of the HBs antigen that had been bound to the particle was dissociated, due to long reaction with the alkali aqueous solution at a high concentration. As a result, the ΔOD × 10000 values greatly decreased, and it was difficult to detect the presence of the anti-HBs mouse antibody. One of the factors is considered that the HBs antigen was denatured due to the alkali aqueous solution.

As can be seen in Table 1, the particle size of the particle for immunoassay for detecting an anti-HBs antibody treated by the reductant (dithiothreitol) slightly increased, the ΔOD × 10000 values greatly decreased, and it was difficult to detect the presence of the anti-HBs mouse antibody. The reason is considered that the reductant destroys the nearby structure of the antigenic determinant a, which is a main conformational epitope of the HBs antigen, the anti-HBs mouse antibody could not bind thereto.

On the other hand, the particle for immunoassay for detecting an anti-HBs antibody treated by the acidic aqueous solution (hydrochloride) had slightly smaller particle size (Table 1) and increased ΔOD × 10000 values (Table 2) compared to the reference. The reason is considered that, in contrast to the alkali aqueous solution or reductant, because the acidic aqueous solution suitably reacted to the HBs antigen sensitized on the particle surface and exerted the effect of removing impurities, and their sensitivity improved.

### (Example 6: Real Experimental Evaluation of Sensitivity to Anti-HBs Antibody)

Next, the sensitivity of the particle for immunoassay for detecting an anti-HBs antibody prepared in Example 3, which was reacted with 10% aqueous solution of CHAPSO, and the particle for immunoassay for detecting an anti-HBs antibody prepared in Comparative Example 2, which as mixed with 10 mM HEPES buffer solution (pH 7.9) was evaluated by the latex immunoagglutination method using a clinical testing apparatus, TBA-120FR (manufactured by CANON MEDICAL SYSTEMS CORPORATION). The concentration of the anti-HBs antibody in the serum of human anti-HBs antibody positive patients were previously measured, and the mixture was then diluted with human anti-HBs antibody negative serum so that the concentrations of the anti-HBs antibody be 0, 200 and 1000 mIU/mL. The solution mixing conditions in measurement were set to 35 µL each of the above described serum, 25 µL buffer solution for diluting specimen (PBS, 0.01 wt% Tween (registered trademark) 20, 0.5% PEG500K) and 20 µL dispersion solution of the particle for immunoassay for detecting an anti-HBs antibody (particle concentration of 0.3 wt%, 10 mM HEPES, pH 7.9, 0.01 wt% Tween (registered trademark) 20). The absorbance was measured at 572 nm according to general measurement protocol. Specifically, the mixed solution of the serum and the buffer solution for diluting specimen was heated at 37°C for 5 minutes, the dispersion solution of the particle for immunoassay for detecting an anti-HBs antibody was then added to measure the values of absorbance at 572 nm at a time immediately after the addition and 5 minutes after the addition.

The value obtained by subtracting the absorbance immediately after the addition from the absorbance 5 minutes after the addition was calculated and multiplied by 10000 to obtain a ΔOD × 10000 value.

The results are shown in FIG. 1. The ΔOD × 10000 value of the particle for immunoassay for detecting an anti-HBs antibody reacted with the aqueous solution of CHAPSO increased compared to the particle for immunoassay for detecting an anti-HBs antibody mixed with the 10 mM HEPES buffer solution (reference).

Also, the particle for immunoassay for detecting an anti-HBs antibody of the reference did not have a significant difference in ΔOD × 10000 values between the anti-HBs antigen antibody concentrations of 0 mIU/mL and 200 mIU/mL. On the other hand, the particle for immunoassay for detecting an anti-HBs antibody reacted with the aqueous solution of CHAPSO had dominantly increased ΔOD × 10000 value at the anti-HBs antibody concentration of 200 mIU/mL compared to that at the anti-HBs antibody concentration of 0 mIU/mL, indicating that 0 mIU/mL and 200 mIU/mL are distinguishable. In other words, the production method of the present invention can measure the anti-HBs antibody at a lower concentration. The reason is considered that the aqueous solution of CHAPSO suitably reacted to the HBs antigen-sensitized particle sensitized on the particle surface and exerted the effect of removing impurities, and their sensitivity improved.

### (Comparative Example 3)

0.125 mL aqueous solution of 1 mg/mL HBs antigen, 0.1 mL aqueous solution of 10% dodecyl-β-D-maltoside and 0.275 mL PBSO were mixed and shook at 25°C for 24 hours.

After that, purification and concentration were performed by dialysis and Amicon Ultra (manufactured by Merck) centrifugal ultrafiltration filter. The thus obtained HBs antigen was used to sensitize to the particle in the same manner as in Example 2, and as a result, the amount sensitized remained only at 33 µg/mg, and the sensitization efficiency was poor. The reason is considered that the surfactant remains in the HBs antigen solution. The reason is considered that the surfactant slightly remained even after the dialysis and purification were thoroughly performed hinders the sensitization reaction (binding reaction) with the particle.

Further, the particle for immunoassay for detecting an anti-HBs antibody was used to evaluate the sensitivity to the anti-HBs antibody in the same manner as in Example 5, and as a result, ΔOD × 10000 value was 60 also at the anti-HBs mouse antibody concentration of 1 mg/mL. From the above, it can be said that the treatment with surfactant or the like before covalently binding to the particle does not contribute to the improvement in detection sensitivity of the anti-HBs antibody.

### (Example 7: Synthesis of Polystyrene Particle Containing Siloxane)

90 g phosphate buffer solution (manufactured by Kishida Chemical Co., Ltd., pH 7.4) was loaded into 200 mL flask, and to this solution, 0.9 g polyvinylpyrrolidone K-30 (manufactured by Kishida Chemical Co., Ltd., the molecular weight of 40000) was dissolved. Next, 3.0 g 3-methacryloxypropyltrimethoxysilane (trade name: LS-3380; manufactured by Shin-Etsu Chemical Co., Ltd.) and 9.0 g styrene (manufactured by Kishida Chemical Co., Ltd.) were loaded to the flask to stir the mixture while blowing nitrogen at room temperature for 10 minutes. Emulsion in the flask was then heated to 70°C in an oil bath. A solution obtained by dissolving 0.3 g potassium peroxodisulfate (manufactured by Wako Pure Chemical Industries, Ltd.) in 15 mL phosphate buffer solution (manufactured by Kishida Chemical Co., Ltd., pH 7.4) was added to the emulsion heated to 70°C. After stirring for 7 hours held at 70°C, the temperature was cooled to room temperature to obtain a dispersion of a microparticle of polystyrene-silica hybrid.

To the thus obtained dispersion of a microparticle of polystyrene-silica hybrid, 2 g Tween 20 (manufactured by Tokyo Chemical Industry Co., Ltd.) was added as the surfactant, and 0.1 mL X-12-1135 (manufactured by Shin-Etsu Chemical Co., Ltd.), which is a silane coupling agent having a carboxy group, was further added to stir the mixture at room temperature for 14 hours. By this, a carboxylated polystyrene-silica hybrid microparticle was obtained.

### (Example 8: Sensitization of HBs Antigen, and Processing Operation with Surfactant or the Like)

Sensitization of HBs antigen and processing operation with surfactant or the like were performed to the carboxylated polystyrene-silica hybrid microparticle in the same manner as in Examples 2 to 4.

### (Comparative Example 3)

Preparation was performed to the HBs antigen-sensitized carboxylated polystyrene-silica hybrid microparticle as in Example 8 except that 0.84 mL 10 mM HEPES buffer solution (pH 7.9) was used in place of the surfactant used in Example 8 or the like to obtain a particle for immunoassay for detecting an anti-HBs antibody.

The properties of the particles for immunoassay for detecting an anti-HBs antibody prepared in Example 8 and Comparative Example 3 are shown in Table 3.

**[Table 3]**

| | Item | Name | Volume average particle size (nm) | Poly dispersity index | Amount sensitized per 1 mg of particle (µg/mg) |
|---|---|---|---|---|---|
| Example 8 | Nonionic surfactant | Dodecyl-β-D-maltoside | 225.9 | 0.01 | 90 |
| | Nonionic surfactant | Triton(registered trademark)X-100 | 226.3 | 0.004 | 91 |
| | Amphoteric surfactant | 3-[(3-cholamidopropyl)dimethylammonio]-2-hydroxy-1-propanesulfonate | 223.8 | 0.012 | 91 |
| | Acidic aqueous solution | Hydrochloric acid | 267 | 0.098 | 88 |
| Comparative example 3 | Reference | HEPES buffer solution | 236.5 | 0.02 | 98 |

### (Example 9: Evaluation of Sensitivity to Anti-HBs Antibody)

Evaluation of sensitivity to the anti-HBs antibody was performed in the same manner as in Example 5. The results are shown in Table 4.

**[Table 4]**

| | Item | Name | ΔOD × 10000 | | Increase/decrease rate to reference at anti-HBs antibody concentration of 0.1 mg/mL |
|---|---|---|---|---|---|
| | | | Anti-HBs antibody concentration of 0 mg/mL | Anti-HBs antibody concentration of 0.1 mg/mL | |
| Example 8 | Nonionic surfactant | Dodecyl-β-D-maltoside | 110 | 7770 | 1.2 |
| | Nonionic surfactant | Triton(registered trademark)X-100 | 220 | 7500 | 1.2 |
| | Amphoteric surfactant | 3-[(3-cholamidopropyl)dimethylammonio]-2-hydroxy-1-propanesulfonate | 190 | 7100 | 1.1 |
| | Acidic aqueous solution | Hydrochloric acid | -150 | 6900 | 1.1 |
| Comparative example 3 | Reference | HEPES buffer solution | 70 | 6500 | 1.0 |

The particles for immunoassay for detecting an anti-HBs antibody treated by the nonionic surfactant, the amphoteric surfactant, the acidic aqueous solution (hydrochloride) had greatly increased ΔOD × 10000 values (Table 4) compared to the reference. The reason is considered that these surfactants and the acidic aqueous solution suitably reacted to the HBs antigen-sensitized particle sensitized on the particle surface and exerted the effect of removing impurities, and their sensitivity improved.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A method for producing a particle for immunoassay for detecting an anti-HBs antibody, **characterized by** comprising a first step of covalently binding an HBs antigen to a particle to obtain an HBs antigen-sensitized particle, and a second step of washing the HBs antigen-sensitized particle with a nonionic surfactant or an amphoteric surfactant to obtain a washed HBs antigen-sensitized particle.

2. A method for producing a particle for immunoassay for detecting an anti-HBs antibody, **characterized by** comprising a first step of covalently binding an HBs antigen to a particle to obtain an HBs antigen-sensitized particle, and a third step of washing the HBs antigen-sensitized particle with an acidic aqueous solution to obtain a washed HBs antigen-sensitized particle.

3. The production method according to claim 1, comprising a fourth step of further replacing a dispersion solution of the washed HBs antigen-sensitized particle with a buffer solution.

4. The production method according to claim 2, comprising a fourth step of further replacing a dispersion solution of the washed HBs antigen-sensitized particle with a buffer solution.

5. The production method according to claim 1, wherein the covalent bond is an amide bond between an amino group of the HBs antigen and a carboxy group of the particle.

6. The production method according to claim 2, wherein the covalent bond is an amide bond between an amino group of the HBs antigen and a carboxy group of the particle.

7. The production method according to claim 5, wherein the particle is a styrene-glycidyl (meth)acrylate copolymer, and one or more glycidyl groups are ring-opened with mercapto succinate and 3-mercapto-1,2-propanediol.

8. The production method according to claim 6, wherein the particle is a styrene-glycidyl (meth)acrylate copolymer, and one or more glycidyl groups are ring-opened with mercapto succinate and 3-mercapto-1,2-propanediol.

9. The production method according to any one of claims 1, 3, 5 or 7, wherein the nonionic surfactant is one or more selected from the group of dodecyl-β-D-maltoside and Triton X-100, or the amphoteric surfactant is selected from CHAPSO.

10. The production method according to any one of claims 2, 4, 6 or 8, wherein the acidic aqueous solution is one or more selected from the group of an aqueous solution of hydrochloric acid, an aqueous solution of acetic acid and an aqueous solution of glycine hydrochloride.

11. A particle for immunoassay for detecting an anti-HBs antibody, produced by a production method **characterized by** comprising a first step of covalently binding an HBs antigen to a particle to obtain an antigen-sensitized particle, and a second step of washing the antigen-sensitized particle with a nonionic surfactant or an amphoteric surfactant to obtain a washed HBs antigen-sensitized particle.

12. A particle for immunoassay for detecting an anti-HBs antibody, produced by a production method **characterized by** comprising a first step of covalently binding an HBs antigen to a particle to obtain an antigen-sensitized particle, and a third step of washing the antigen-sensitized particle with an acidic aqueous solution to obtain a washed HBs antigen-sensitized particle.

13. The particle for immunoassay for detecting an anti-HBs antibody according to claim 11, wherein the production method comprises a fourth step of further replacing a dispersion of the washed HBs antigen-sensitized particle with a buffer solution.

14. The particle for immunoassay for detecting an anti-HBs antibody according to claim 11, wherein the covalent bond is an amide bond between an amino group of the HBs antigen and a carboxy group of the particle.

15. The particle for immunoassay for detecting an anti-HBs antibody according to claim 14, wherein the particle is a styrene-glycidyl (meth)acrylate copolymer, and one or more glycidyl groups are ring-opened with mercapto succinate and 3-mercapto-1,2-propanediol.
